(19) Europäisches Patentamt
European Patent Office
Office européen des brevets

(11) **EP 4 292 470 A1**

(12) # EUROPEAN PATENT APPLICATION

(43) Date of publication:
**20.12.2023 Bulletin 2023/51**

(21) Application number: **22178869.8**

(22) Date of filing: **14.06.2022**

(51) International Patent Classification (IPC):
**A46B 13/02** $^{(2006.01)}$    **A46B 15/00** $^{(2006.01)}$

(52) Cooperative Patent Classification (CPC):
**A46B 13/02; A46B 15/0006;** A46B 2200/1066

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
- **VAN LEEUWEN, Marinus Bastiaan
  Eindhoven (NL)**
- **VLUTTERS, Ruud
  Eindhoven (NL)**

(74) Representative: **Philips Intellectual Property &
Standards
High Tech Campus 52
5656 AG Eindhoven (NL)**

(54) **A LOCATION SENSING SYSTEM FOR A PERSONAL CARE OR HEALTH DEVICE**

(57)    A location sensing system is provided for a personal health or personal care device, for sensing a location of the personal health or personal care device during a use session. Motion information from an IMU for a current use session, up to a current point in time, is processed together with historical data relating to previous use sessions, comprising location information over time for those previous use sessions. The historical data is based on post-session offline analysis of the motion information of those previous use sessions. A current location is derived in real-time based on the motion information and the historical data.

FIG. 2

EP 4 292 470 A1

**Description**

FIELD OF THE INVENTION

This invention relates to location sensing systems, and in particular to location sensing of handheld devices, such as personal health or personal care devices.

BACKGROUND OF THE INVENTION

Many handheld devices are equipped with inertia monitoring unit (IMU) sensors to monitor their movement and location. A typical example is mobile phones. However, there is also an increasing use of location (i.e. position) sensing in personal care or personal health or personal care devices such as electronic toothbrushes and shavers. For such devices, the IMU data is used to localize where the user is applying the device.

As an example, location sensing can be used to determine the tooth segment (i.e. adjacent group of teeth) the user is currently brushing, which enables feedback to the user about the quality of their brushing session, in terms of the time spent on the different tooth segments. With this feedback, users can improve their daily brushing routine.

IMU sensors only give movement information rather than absolute location information. To determine absolute location information, there needs to be a known starting position, from which movement can then be tracked using the IMU movement information, thereby to track changes in position.

For a toothbrush, the location information of interest is the tooth segment at which the user is brushing as mentioned above, so that a report can be provided of how well different areas were brushed. To detect in which segment a user is brushing based on the toothbrush IMU signals is a challenging problem, as only the toothbrush motion is observed, but it is the relative motion between the brush and the teeth that is of interest. By measuring motion of the head, when users are walking around their bathroom, this will result in additional signals that the algorithm (e.g. an AI model) has to learn to ignore.

Furthermore, (in theory) it is possible to brush your teeth by keeping the toothbrush static (or almost static) and moving your head and mouth around the brush. In this case, there will hardly be any acceleration signals measured by the toothbrush IMU, hence it will be very difficult or nearly impossible to detect the segments.

Nevertheless, brushing in certain segments (and their transitions) are typically done with specific motions, so the resulting IMU signals can be used to reason where the brush was in the mouth. For example, it is known to detect motion patterns and using these detected patterns, the segment location can be deduced in the complete brushing session.

There are in principle two ways to analyze IMU sensor signals:

offline, where all IMU sensor data is recorded, and upon completion of the session, the complete session is analyzed as one; and

real-time (online), where IMU sensor data is continuously used by an algorithm to provide predictions during the session.

Based on offline analysis, there are existing toothbrushes that can determine after a brushing session how much time the user has spent brushing in each of the different (e.g. 12) tooth segments. Furthermore, the algorithm may also calculate pressure and scrubbing maps for each segment.

With real-time analysis, a new smarter toothbrush can be built. Based on the real-time location, the toothbrush motor may be controlled, or direct user feedback may be provided (with for example lights or sound). Although real-time assessment enables valuable features, it is more difficult than offline analysis, hence it typically results in lower localization accuracy.

For an offline algorithm, the prediction of the brushing segment at time T is based on IMU data from the complete brushing session (t=[0..Tend]), hence using data from before and after time T. In a real-time model, sensor data can only be used from the past, hence from t=[0..T].

Additionally, a real-time model typically needs to run inside the device, where cost and power constraints limit the available computer hardware. An offline model typically runs on a separate device, e.g. a mobile phone or even in the cloud. Therefore, offline models can be of much higher computing complexity than real-time models, resulting in a higher accuracy.

There is therefore a need to improve the accuracy of real-time localization, in particular to reduce the accuracy gap between offline and real-time models.

SUMMARY OF THE INVENTION

The invention is defined by the claims.

According to examples in accordance with an aspect of the invention, there is provided a location sensing system for a personal health or personal care device, for sensing a location of the personal health or personal care device during a use session, comprising:

an inertia monitoring unit for providing motion information related to motion of the personal health or personal care device; and

a processor for processing the motion information to derive location information, wherein the processor is configured to:

receive motion information for the current use session, up to a current point in time;

receive historical data relating to previous use sessions comprising location information over time for previous use sessions based on post-session offline analysis of the motion information of those previous use sessions; and

determine a current location in real-time based on the motion information and the historical data.

This system combines the flexibility of real-time location sensing with the accuracy of offline post-session analysis. This offline post-session analysis makes use of an algorithm which derives location from both previous and future movement information of a session and thus reconstructs location data more accurately than real-time analysis. By combining this offline analysis with real-time motion sensing, more accurate real-time location sensing is possible.

The real-time analysis has only current and previous motion sensing signals, and with this analysis alone, the location can be ambiguous such that there are various possible locations. Only by reasoning over a complete session (which must therefore be performed offline) is it possible to determine the correct location and hence reduce the ambiguity. This ambiguity is reduced by injecting the predictions from the offline model. This works generally for personal health devices or personal care devices where users perform sessions in a consistent and repetitive manner, e.g. a fixed tooth brushing or shaving routine.

The system in particular may be operated such that the real-time performance gradually improves by use of an offline model, which is easier to train over time. The real-time model is typically simpler than the offline model, which matches with the amount of computing power available in the device (in real-time) versus the offline model (offline, in an app or in the cloud). The term offline is simply used to mean not in real-time, and hence with the option to freely choose where and when to perform the processing since a delay in processing is not an issue.

The historical data for example comprises an averaging of a determined location over time for multiple previous use sessions. Thus, it represents an average location sequence. It may be considered to represent a probability of a particular location, or the location for which the probability is highest.

A determined location for example comprises a location class which is one of a set of location classes. These classes are for example location ranges with a shared significance, such as all locations corresponding to a tooth or to a set of teeth forming a particular tooth segment.

If the location sensing system is for a shaver, each location class for example corresponds to a face region.

The processor is for example configured to update the historical data after a current use session is complete, based on post-session offline analysis of the motion information of the current use session. Thus, a more accurate analysis of a current session (using the offline model) may be used to update the historical data. This update may be performed after each use session or less frequently.

The processor is for example configured to update the historical information using a moving average update. This updates the historical session information by combining the last current session information (after offline analysis) with the already existing historical session information.

The inertia monitoring unit for example comprises a three-axis accelerometer and a three-axis gyroscope. Thus, six dimensional motion data is collected.

A personal health or personal care system may be provided which combines a personal health or personal care device, and the location sensing system defined above, and a computer program for performing offline post-session analysis of the motion information of previous use sessions and generate the historical data relating to those previous use sessions. The computer program is run on a remote device, separate to the personal health or personal care device, and the remote device and the personal health or personal care device are able to communicate with each other.

The invention also provides a powered toothbrush comprising:

a toothbrush handle having an interface for connecting to a toothbrush head;

a drive motor for driving the interface for driving the toothbrush head; and

the location system defined above for determining the location of the toothbrush head during a use session.

The invention also provides an electric shaver comprising:

a shaver head;
a drive motor for driving the shaver head; and
the location system defined above for determining the location of the shaver head during a use session.

The invention also provides a method for determining a location of a personal health or personal care device during a use session, comprising:

receiving motion information relating to motion of the personal health or personal care device for the current use session, up to a current point in time;
receiving historical data relating to previous use sessions comprising location information over time for previous use sessions based on post-session offline analysis of the motion information of those previous use sessions; and
determining a current location in real-time based on the motion information and the historical data.

The historical data is based on offline analysis (rather than simply a storage of the real-time analysis from previous sessions) so that it increases the accuracy of the real-time analysis more than simply using historical real-time analysis. The real-time analysis is for example performed using local processing power of the device whereas the offline analysis is performed remotely.

The method for example comprises deriving the historical data by averaging determined locations over time for multiple previous use sessions. The real-time sensor data from the current session is in this way mixed with the average from previous sessions. This avoids the need for a user to be extremely consistent with their personal care routine. The historical data provides an expected set of locations over time.

The method for example comprises updating the historical data after a current use session is complete based on post-session offline analysis of the motion information of the current use session. This updating for example makes use of a moving average update.

The invention also provides a computer program comprising computer program code means which is adapted, when said program is run on a computer, to implement the method defined above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a location system applied to a powered toothbrush;
Figure 2 shows an offline assessment algorithm;
Figure 3 shows how historical data is updated;
Figure 4 shows a real-time assessment algorithm;
Figure 5 shows typical IMU signals during a complete brushing session;
Figure 6 shows identified brushing segments overtime based on the data of Figure 5;
Figure 7 shows a model for offline assessment;
Figure 8 shows a model for real-time assessment;
Figure 9 shows four examples of different user sessions (averaged over multiple sessions of each user);
Figure 10 shows segment probabilities as a function of time averaged for all users and all sessions within a database; and
Figure 11 shows a location determination method.

DETAILED DESCRIPTION OF THE EMBODIMENTS

The invention will be described with reference to the Figures.

It should be understood that the detailed description and specific examples, while indicating exemplary embodiments of the apparatus, systems, and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a location sensing system for a personal health or personal care device, for sensing a location of the personal health or personal care device during a use session. Motion information from an IMU for a current use session, up to a current point in time, is processed together with historical data relating to previous use sessions, comprising location information over time for those previous use sessions. The historical data is based on post-session offline analysis of the motion information of those previous use sessions. A current location is derived in real-time based on the motion information and the historical data. This approach can avoid the need for intermediate motion pattern detections.

As explained above, offline assessment of location is easier because all data of the session can be used for the interpretation of the movement, but the findings of the analysis only become available after the session has been completed. For the example of providing tooth brushing performance analysis, it is only possible at best to tell the user based on this information that he/she should have brushed in a different way real-time analysis has the advantage that it enables instant feedback to the user.

For real-time analysis, only samples of the session until current time t can be used, and no information of future samples in the session are available for the interpretation. This is a well-known technical problem of real-time assessment.

For the example of tracking location of a toothbrush head, at the start of a brushing session, the inertia monitoring unit (IMU) data doesn't provide any information about the location in the mouth. It only contains information about the orientation of the brush head (the orientation is known in absolute terms because of the known gravity vector). If the orientation of the brush head is facing upwards, it may be deduced that the user started to brush the chewing surface of the upper teeth, but it doesn't contain information about the horizontal position in the upper jaw. Clues about the horizontal position can only be derived from future samples of the session. For example, if the future samples indicate a significant horizontal movement of the brush head to the left, then it can be concluded that the user started somewhere on the right side of the jaw.

This example illustrates how the assessment of the location of the brush head at time t benefits from availability of session data recorded after time t. This "future" data is available in case of offline assessment but is not available for real-time assessment.

By way of example, two algorithms for localization were trained using the same IMU datasets. An offline algorithm reached 84.0% test accuracy compared to an accuracy of a real-time assessment of 75.7%.

A well-known compromise between real-time and offline analysis is to perform the analysis with a short delay $\Delta t$. In this case, the analysis can be done on slightly more data (until $t+\Delta t$), but the feedback to the user will also be delayed with the same amount. This delay is additional to the delay of computation time of the algorithm.

The invention significantly improves the accuracy of the real-time assessment without introducing the delay $\Delta t$. Only data that is available at time t is used.

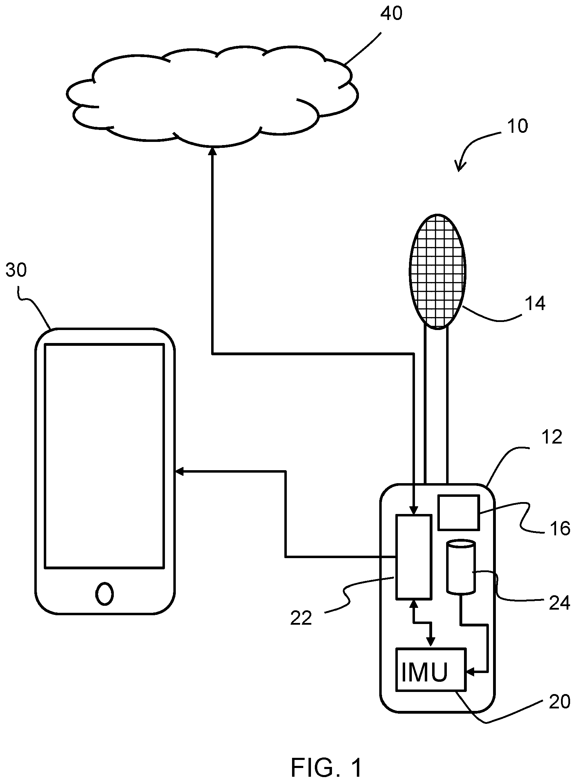

Figure 1 shows the location system of the invention applied to a powered toothbrush.

The powered toothbrush 10 comprises a toothbrush handle 12 having an interface for connecting to a toothbrush head 14. The handle includes a drive motor 16 for driving the interface for driving the toothbrush head.

A location sensing system is used determining the location of the toothbrush head during a use session. The location sensing system comprises an inertia monitoring unit 20 for providing motion information related to motion of the toothbrush.

The inertia monitoring unit for example comprises a three-axis accelerometer and a three-axis gyroscope.

A processor 22 is provided as part of the toothbrush for processing the motion information to derive location information.

The processor receives motion information for the current use session, up to a current point in time, from the IMU. However, it also receives historical data relating to previous use sessions comprising location information over time for previous use sessions based on post-session offline analysis of the motion information of those previous use sessions. This data is stored in database 24. However, it is preferably generated remotely rather than using the processor 22.

This processing may for example be carried out by the processor of a mobile phone 30 or in the cloud 40. The toothbrush for example sends data to the mobile phone 30 which acts as a user interface to the toothbrush, and it is used to convey information to the user via an app on the mobile phone.

A current location is determined in real-time based on the motion information and the historical data. The system thus uses two inputs. One is the usual IMU sensor data of the current session up to current time t, and the other is historical data that relates to information that has been extracted from historical sessions.

The solution thus makes use of two separate analysis algorithms:

an offline part creates the historical data hd[0..t] for each user (which is function of time);
a real-time part that derives a real-time assessment from the IMU input IMU[0..t] and the historical data hd[0..t].
[0..t] specifies the time interval of the data from beginning of the session (t=0) to the current time t.

The purpose of the offline part is to capture information about the device movements that have been observed in previous sessions. The basis for the offline part is a more accurate offline assessment algorithm. This algorithm is applied to complete sessions that have been collected in the past. This analysis yields prediction data about the expected location over time during a session. The prediction data for all prior sessions of the user are for example accumulated and averaged over time to generate the historical data hd.

The historical data hd is a 2-dimensional data array of size n_time_samples (rows) x n classes (columns). Thus, for each time sample, a value is allocated to each location class, in particular a probability value.

For the application of localization of the toothbrush head, the mouth is for example subdivided into 12 segments, so that n_classes = 12. Thus, the determined location is not recorded as an absolute location but is allocated to a location class which is one of a set of location classes.

The value n_time_samples corresponds to the length of the longest recorded session, i.e. the greatest number of time samples, or a default maximum number of time samples.

The historical data is initialized with data from sessions from many users (e.g. the users in a total training set). After deployment of the algorithm in the device, the historical data can be updated with data from new recordings of the user of the device, but again using the accurate offline assessment algorithm.

By updating the historical data, the information becomes more personal and relevant for the specific user which results in improved performance of the real-time assessment.

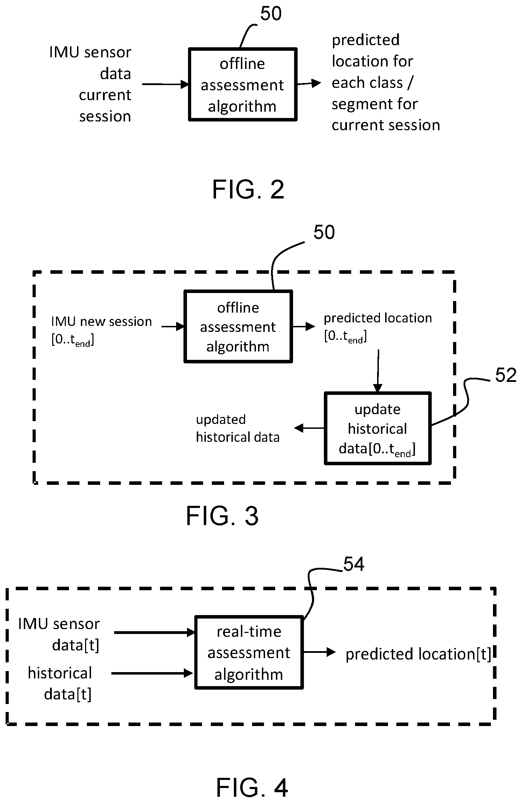

Figure 2 shows the offline assessment algorithm 50. The input of the algorithm is the IMU sensor data of a complete session. The offline assessment algorithm is used to compute the probabilities for each location class for the session. The output is thus the predicted probabilities for each location class for the complete input session.

Figure 3 shows how these probabilities are added to the historical data. An update function is represented by module 52.

Figure 4 shows the real-time assessment algorithm 54. The updated historical data is used as input for the real-time assessment. The input to the algorithm is the IMU sensor data of a session and the historical data, at the current time t. The output is the predicted location at time t.

With this approach, there is no need to ask the user to provide his/her starting position and brushing order, as is currently done for guided brushing using some existing systems.

The approach will now be described in more detail.

The algorithm for offline assessment of a complete session starts from IMU data of a complete session, in the form of a 2-dimensional array with shape $[0...t_{end}, 0..nb_{channels}]$ containing typically $nb_{channels}=6$ channels corresponding to the x/y/z components of the accelerometer and gyroscope signals for $t_{end}$ time steps. Thus, the data from the IMU sensor is time series data, and the accelerometer and gyroscope measure their components in x, y and z directions resulting in 6 channels sampled at around 30 Hz. Given a typical brushing session of about 2 minutes this results in a matrix of ~3600 x 6 values.

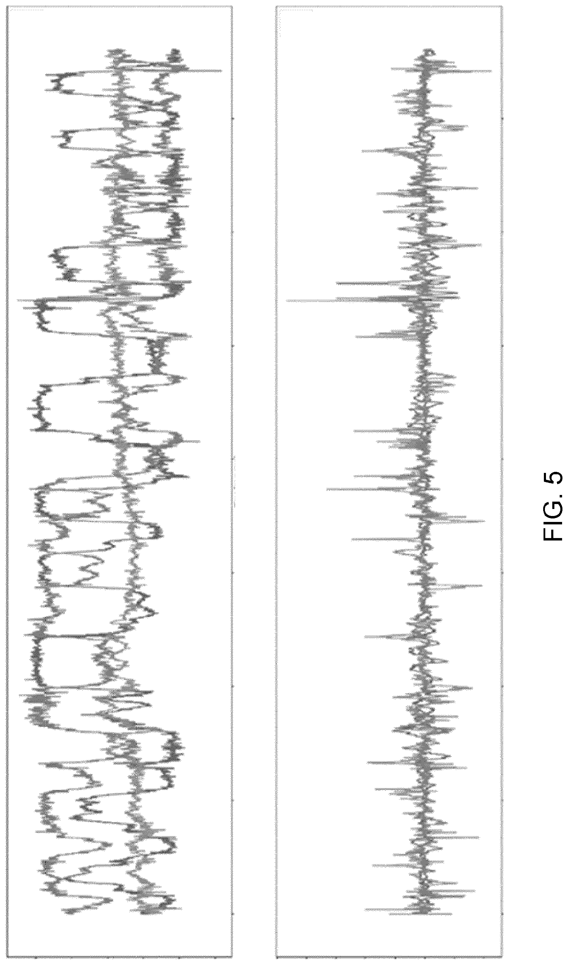

Figure 5 shows typical IMU signals during a complete brushing session. The top graph shows x, y and z components from the accelerometer. The bottom graph shows the x, y and z components from the gyroscope.

The offline model predicts the probability that a user is brushing in segment i for each time step, hence:

$$p_{offline} = M_{offline}(x)$$

Where $p_{offline}$ is a 2 dimensional array with shape $[t_{end}, nb_{segment}]$. x denotes the date for a complete brushing session.

As the offline model can be complex and might require a large amount of memory (>1MB), it will typically be running on the mobile phone or in the cloud. As such it can be updated without updating the real-time model. The performance of the real-time model becomes better for better performing offline models.

The historical data *hd,* is a 2-dimensional array with shape $[t_{end}, nb_{segment}]$ and contains the average probabilities from previous sessions. The historical data will start with an initial version which is not yet tailored to the particular user. As the initial version of the historical data, the average segment probabilities may be used that are predicted by the offline model over a large dataset of brushing sessions (i.e. the training data).

As all sessions have a different length, only a predetermined time period may be used such as a 2 minute time period. This may be the first 2 minutes, as most people brush this advised duration. This then defines the maximum number of time steps. Alternatively, any number of time steps may be permitted, but the historical data for the last time step can be repeated when brushing for longer than the predetermined time period.

Starting from the initial historical data, it can be updated and personalized by calculating an exponential moving average update using the probabilities from the offline model:

$$hd_{new} = (1 - \alpha) \cdot hd_{old} + \alpha \cdot p_{offline}$$

It is not necessary that each new prediction of the offline model directly results in an update of the historical data. Multiple offline predictions can also be combined in a single update of the historical model.
The exponential multiplier $\alpha$ is for example in the range 0.5 to 0.05.
The real-time model takes both the current IMU sample $x[t]$ as well as the historical data of that same moment $hd[t]$ as input, and predicts the current segment probabilities $p[t]$, hence:

$$p_{online}[t] = M_{real-time}(x[t], hd[t])$$

Thus, in this example, only data for a single time point from the historical data is needed at any particular time to perform the real-time processing. Thus, the computational overhead of incorporating the historical data is minimal. A single data set is used indicating the probabilities of being in the different segments at that particular time.
The historical data enables the real-time algorithm to make a selection (between tooth segments) when there is doubt. Thus, the historical data guides (i.e. biases) the decisions taken by the simpler real-time model (without historical data).
When implementing the model ($M_{real-time}$) using a long-short term memory (LSTM), one typically needs to update the internal/hidden state for every time step. The LSTM uses this hidden state to provide context and consistency over the multiple time steps. Therefore, the model obtains one more inputs (containing the previous hidden state) and one more outputs (the updated hidden state), hence the model actually has 3 inputs and 2 outputs:

$$P_{online}[t], hidden[t] = M_{online}(x[t], hd[t], hidden[t-1])$$

At $t$=0, the hidden state is typically initialized with zeros.
The hidden state is a form of representation of the the current state. As such it includes (in an abstract representation) information about the current location as well as the speed and accelerations of the device and possibly deduced head/body motion information.

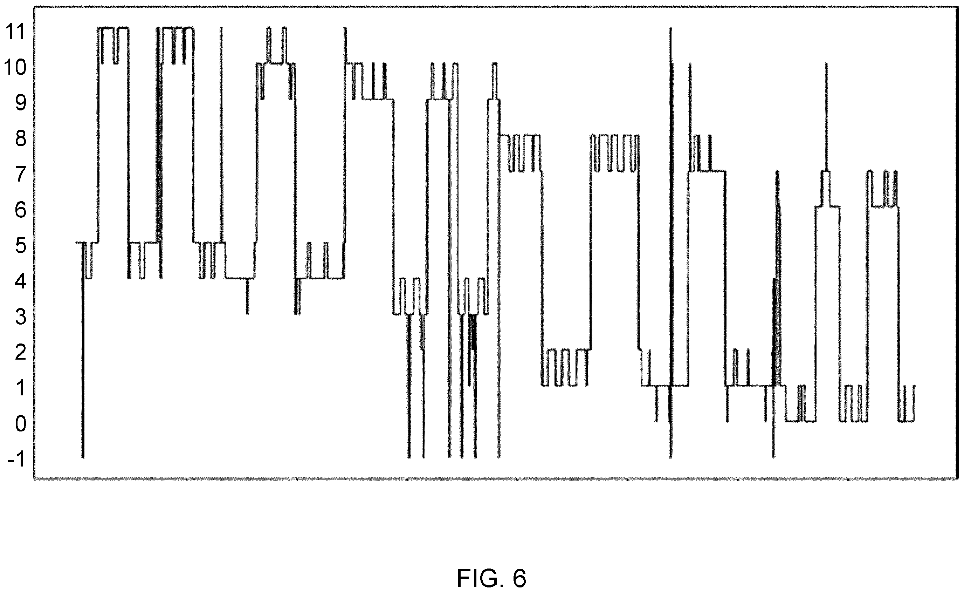

Figure 6 shows the identified brushing segments over time based on the data of Figure 5. Different positions on the y-axis denote the 12 separate segment identities (i.e. numbered 0 to 11).
The 12 segments are:

0: upper right outer
1: upper front outer
2: upper left outer
3: lower left outer
4: lower front outer
5: lower right outer
6: upper right inner
7: upper front inner
8: upper left inner
9: lower left inner
10: lower front inner
11: lower right inner.

The y-axis counts down from 11 to 0 in the positive y-axis direction.
The -1 lowest value on the y-axis is a value used to indicate that the segment cannot be reliably determined.
Accurate location sensing can be used to measure the performance of the location system in determining where the user is brushing and for algorithm training.
There are two ways to measure (rather than estimate) in which segment a person is brushing. A motion capturing system may be used or a movie can be recorded and manually annotated for every video frame (at 30 Hz). This measured location information can be used as the ground truth data for the training of the algorithm.
Typical human action recognition methods are solved as a classification problem. For training the algorithm, a sliding time window is for example taken from the IMU signal and classified to be according to the majority ground truth label in that window. This defines the input for the algorithm training From the IMU signal, the model predicts the most likely class (e.g. segment identifier) at time t. Different sliding window labeling approaches are possible. One is to use the most frequent label over the sequence and the other is to use the label at the last timestamp.

The problem with this approach is that windows can be misaligned with the transitions between the ground truth labels. Furthermore, applying such a classifier will require the network to be applied many times, every time with the time sequence shifted by 1, resulting in many duplicate calculations.

A similar problem is encountered in computer vision algorithms when segmenting images (also called semantic segmentation), for which fully convolutional networks have been developed. The labelling is provided for every time step, so it is possible to predict the labels for all the time steps simultaneously. For this purpose, an AI model or network may be used that will receive as input [nb_time_step x 6] values and will predict as output [nb_time_step x 12] class probability values.

The 6 inputs correspond to the number of channels in the IMU signal, (x, y and z components of the accelerometer and gyroscope signals). The 12 outputs correspond to the number of teeth segments being predicted, thus the number of classes and segment identifiers in Figure 6.

This network will be quite complex, as the correct segment cannot be deduced given only data from a short time window around that moment. For example, in order to deduce if the user starts at the front top or front bottom teeth, data is needed including a flip from top-to-bottom or bottom-to-top so that this information can be used to correctly predict the starting position. Effectively this means that the model should be able to properly model long range/time dependencies.

In one example, the offline model is based on a so-called U-Net that is modified to work on time series data.

The U-Net has been the go-to network of choice for image segmentation using medical datasets.

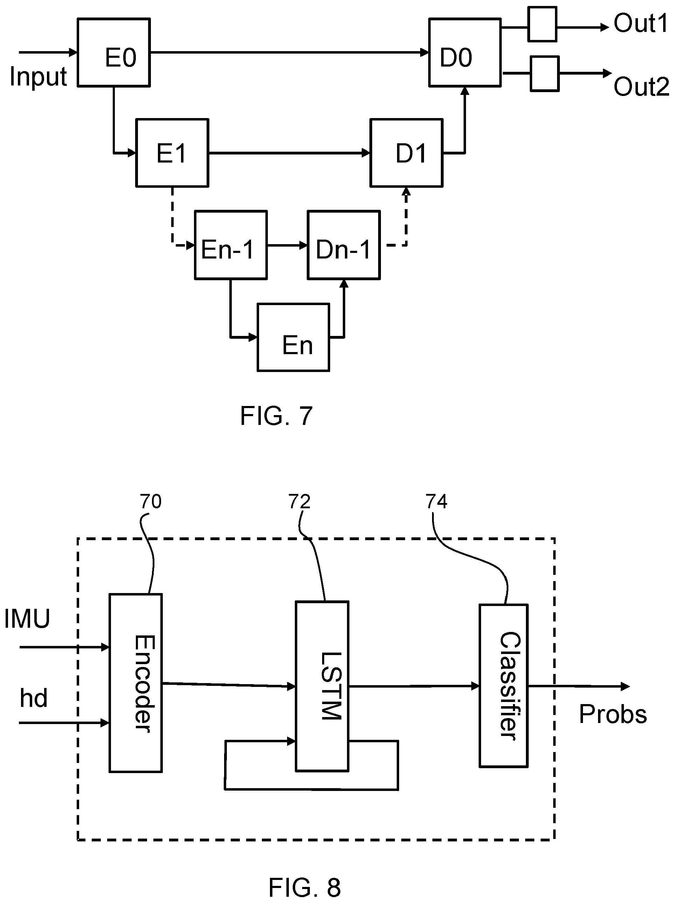

Figure 7 shows a U-Net architecture. In a U-Net the input data is fed through a first encoder (E0), resulting in a first feature representation. Next, this feature representation is down scaled (using max pooling) and fed through a second encoder (E1) resulting in a second feature representation. This process repeats itself several times, resulting in several feature representations at different scales. Next, the lowest resolution feature representation is upscaled (using nearest neighbor up sampling) and combined (concatenated) with the second lowest resolution feature representation and pulled through a decoder.

By repeatedly upscaling, concatenating with the encoder feature representation of the same resolution and pulling the result through the decoder, it is possible finally to obtain a decoded feature representation at the original input resolution.

Two output heads are applied to this final representation (Output 1 and Output 2), one for 12 segment classification and one for position along the tooth-arc regression.

The power of the U-Net comes from its multi-scale analysis of representations, so data is analyzed not only at the original input resolution but also at (factors of 2) smaller resolutions. This enables the efficient modelling of long range dependencies, as long range dependencies will become more local at the lower resolutions. For example, actions taken 17 seconds before or after a certain moment in time, are 17 x 30 = 510 samples apart at the input resolution, but at resolution level 9, they are just 1 time step apart. This makes the modelling of these long range dependencies possible with relatively small convolution kernels.

Additionally, the skip-connections (the horizontal connections from encoder x to decoder x) ensure a strong gradient flow while applying back propagation (used in optimizing the weights), hence U-Nets have less optimization issues compared to other equally deep networks.

For IMU time series data, the convolution layers are modified to be 1-dimensional instead of 2-dimensional.

There are many variants of the U-Net known in literature, most of them keep the same schematic structure as shown in Figure 7 but change the specific layers used in the encoder/decoder blocks.

As an alternative to the U-Net, bi-directional LSTMs may be used. This type of LSTM can only be applied to offline sessions and can reason both forward and backward in time. When training the real-time model with such a bi-directional LSTM instead of a normal LSTM, similar results are obtained to the use of the U-net.

The model for the real-time assessment is for example a conv-LSTM model. As shown in Figure 8 this model consists of 3 parts, an encoder 70, a long short-term memory (LSTM) layer 72 and a classifier 74.

The encoder is made from one or more convolutional layers, that convert the raw IMU signal into a higher dimensional representation. The IMU signal (with its 6 channels) is concatenated with the historical data (hd, with its 12 segments/classes) resulting in an input signal to the encoder with 18 channels. The output of the encoder is used as input to the LSTM.

In the LSTM layer 72, the time-dependent data is processed in time-steps. For each time-step, the input data is combined with the previous hidden state to come to a new updated hidden state and an output.

Finally, in the classifier 74, the output of the LSTM is used as input to a couple of convolutional layers (with kernel size = 1) and rectified linear units (Relu) (so effectively a multi-layer perceptron) to predict the segment probabilities.

As explained above, the historical data consists of the average predictions from previous sessions. The average predictions can be over all users stored in a database.

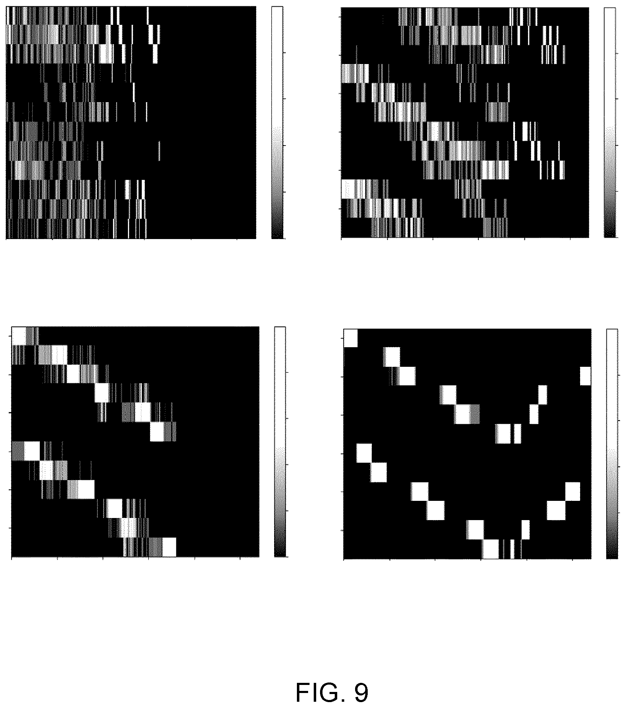

Figure 9 shows four examples of different user sessions (averaged over multiple sessions of each user). In each case, the y-axis has the 12 segments and the x-axis is the time samples (totaling 2 minutes). The grey shading represents probability levels.

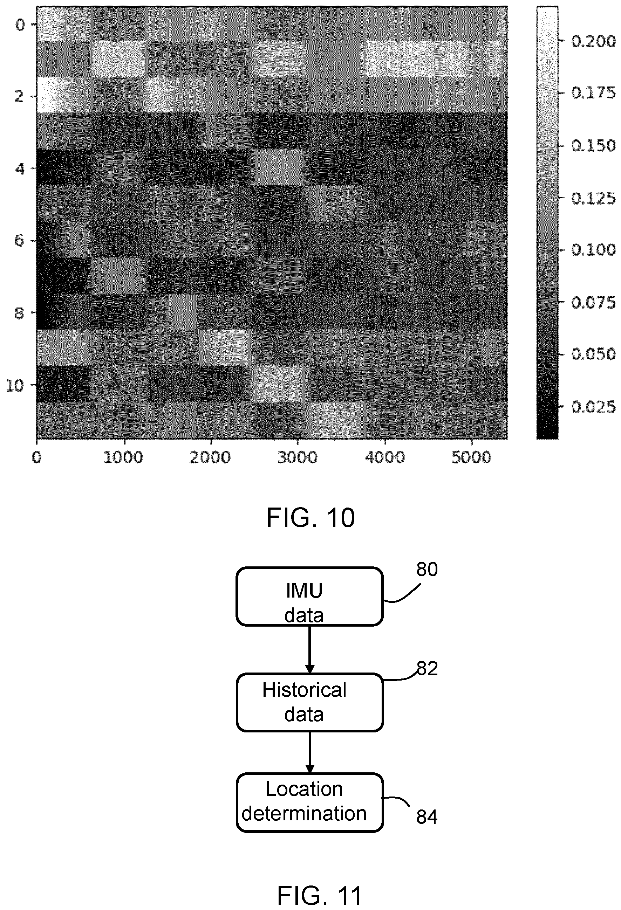

Figure 10 shows segment probabilities as a function of time averaged for all users and all sessions within a database.

From Figure 10, it can be concluded that most people in the database start at segment 2 (hence a high probability near the upper left corner of the plots). Segment 2 thus has the highest probability at t=0. Furthermore, people follow or switch segment in response to a timer buzzer, resulting in the bright and dark blocks. Most people brush for 2 minutes (as this is advised), resulting in strong signals until 120 sec x 30 Hz = 3600 samples.

As each person brushes (and also shaves) in a different order and for a different duration, when only averaging over sessions from the same user, a very different average probability map is obtained (as shown in Figure 9) than the overall average of Figure 10. Some users are very consistent, resulting in very bright blocks as each session perfectly aligns with the previous sessions. Other people are very random in their brushing style, resulting in a more distributed average probability map.

Figure 11 shows a method for determining a location of a personal health or personal care device during a use session. The method comprises, in step 80 receiving motion information related to motion of the personal health or personal care device for the current use session, up to a current point in time. Historical data relating to previous use sessions is received in step 82. It comprises location information over time for previous use sessions based on post-session offline analysis of the motion information of those previous use sessions. A current location is determined in real-time based on the motion information and the historical data in step 84.

The invention has been tested to demonstrate that the location sensing accuracy is improved. The offline model alone yielded 84.0% validation accuracy. A normal real-time model without historical data input yielded 75.7% validation accuracy, and the approach of the invention yielded 81.5% validation accuracy. Each model was trained on the same data, and the validation was carried out on similar data from different users. Thus, the validation accuracy gap of about 8% between an offline and a real-time model is reduced to just below 3%.

The examples above make use of IMU data to track how the user is using the device. Other information may be used, such as information about pressure applied, and button presses, if people use the device in a similar way over different sessions.

The invention is of primary interest for personal health devices, like toothbrushes and shavers. However, it can be used for other user devices. As long as a user is using a device in a user specific, more or less consistent way, the use of historical data can enable improved real-time predictions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner (optional)

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. (optional)

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

**Claims**

1. A location sensing system for a personal health or personal care device (10), for sensing a location of the personal health or personal care device during a use session, comprising:

    an inertia monitoring unit (20) for providing motion information related to motion of the personal health or personal care device; and
    a processor (22) for processing the motion information to derive location information, wherein the processor is configured to:

        receive motion information for the current use session, up to a current point in time;
        receive historical data relating to previous use sessions comprising location information over time for previous use sessions based on post-session offline analysis of the motion information of those previous use sessions; and

determine a current location in real-time based on the motion information and the historical data.

2. The system of claim 1, wherein the historical data comprises an averaging of a determined location over time for multiple previous use sessions.

3. The system of any one of claims 1 to 2, wherein a determined location comprises a location class which is one of a set of location classes.

4. The system of claim 3, wherein the location sensing system is for a toothbrush (10), and wherein each location class corresponds to a tooth segment.

5. The system of any one of claims 1 to 3, wherein the location sensing system is for a shaver, and wherein each location class corresponds to a face region.

6. The system of any one of claims 1 to 5, wherein the processor (22) is configured to update the historical data after a current use session is complete based on post-session offline analysis of the motion information of the current use session.

7. The system of claim 6, wherein the processor (22) is configured to update the historical information using a moving average update.

8. The system of any one of claims 1 to 7, wherein the inertia monitoring unit (20) comprises a three-axis accelerometer and a three-axis gyroscope.

9. A powered toothbrush comprising:

    a toothbrush handle (12) having an interface for connecting to a toothbrush head (14);
    a drive motor (12) for driving the interface for driving the toothbrush head; and
    the location sensing system of any one of claims 1 to 8 for determining the location of the toothbrush head during a use session.

10. An electric shaver comprising:

    a shaver head;
    a drive motor for driving the shaver head; and
    the location sensing system of any one of claims 1 to 8 for determining the location of the shaver head during a use session.

11. A method for determining a location of a personal health or personal care device during a use session, comprising:

    (80) receiving motion information related to motion of the personal health or personal care device for the current use session, up to a current point in time;
    (82) receiving historical data relating to previous use sessions comprising location information over time for previous use sessions based on post-session offline analysis of the motion information of those previous use sessions; and
    (84) determining a current location in real-time based on the motion information and the historical data.

12. The method of claim 11, comprising deriving the historical data by averaging determined locations over time for multiple previous use sessions.

13. The method of claim 11 or 12, comprising updating the historical data after a current use session is complete based on post-session offline analysis of the motion information of the current use session.

14. The method of claim 13, comprising updating the historical information using a moving average update.

15. A computer program comprising computer program code means which is adapted, when said program is run on a computer, to implement the method of any one of claims 10 to 14.

FIG. 1

50

IMU sensor
data
current
session → | offline
assessment
algorithm | → predicted
location for
each class /
segment for
current session

## FIG. 2

50

IMU new session
[0..$t_{end}$] → | offline
assessment
algorithm | → predicted location
[0..$t_{end}$]
↓

52

updated
historical data ← | update
historical
data[0..$t_{end}$] |

## FIG. 3

54

IMU sensor
data[t] →
historical
data[t] → | real-time
assessment
algorithm | → predicted location[t]

## FIG. 4

FIG. 5

FIG. 6

FIG. 7

FIG. 8

FIG. 9

EP 4 292 470 A1

FIG. 10

FIG. 11

18

Europäisches
Patentamt
European
Patent Office
Office européen
des brevets

## EUROPEAN SEARCH REPORT

Application Number

EP 22 17 8869

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/211414 A1 (OGUNSINA TOLULOPE [GB] ET AL) 2 July 2020 (2020-07-02) * paragraphs [0010], [0060], [0061], [0108], [0111], [0130], [0136], [0141], [0169]; figures 1,4 * | 1-15 | INV. A46B13/02 A46B15/00 |
| X | US 2021/393026 A1 (SUBHASH HREBESH MOLLY [US] ET AL) 23 December 2021 (2021-12-23) * paragraphs [0070], [0161], [0178], [0182]; figure 12 * | 1-15 | |
| X | US 2020/179089 A1 (SERVAL THOMAS [FR] ET AL) 11 June 2020 (2020-06-11) * paragraphs [0034], [0169], [0181], [0109], [0191] - [0193]; claim 9 * | 1-15 | |
| X | EP 3 858 288 A1 (KONINKLIJKE PHILIPS NV [NL]) 4 August 2021 (2021-08-04) * paragraphs [0010], [0043], [0051], [0052]; claims 1,4 * | 1-15 | |

TECHNICAL FIELDS
SEARCHED      (IPC)

A46B

The present search report has been drawn up for all claims

| Place of search | Date of completion of the search | Examiner |
|---|---|---|
| The Hague | 29 November 2022 | Kun, Karla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 17 8869

This annex lists the patent family members relating to the patent documents cited in the above-mentioned European search report.
The members are as contained in the European Patent Office EDP file on
The European Patent Office is in no way liable for these particulars which are merely given for the purpose of information.

29-11-2022

| Patent document cited in search report | | Publication date | Patent family member(s) | | Publication date |
|---|---|---|---|---|---|
| US 2020211414 | A1 | 02-07-2020 | EP | 3668346 A1 | 24-06-2020 |
| | | | JP | 2020534879 A | 03-12-2020 |
| | | | US | 2020211414 A1 | 02-07-2020 |
| | | | WO | 2019034854 A1 | 21-02-2019 |
| US 2021393026 | A1 | 23-12-2021 | US | 2021393026 A1 | 23-12-2021 |
| | | | WO | 2021262528 A1 | 30-12-2021 |
| US 2020179089 | A1 | 11-06-2020 | AU | 2017316731 A1 | 21-03-2019 |
| | | | BR | 112019003640 A2 | 21-05-2019 |
| | | | CN | 110213980 A | 06-09-2019 |
| | | | EP | 3493701 A1 | 12-06-2019 |
| | | | RU | 2019106205 A | 22-09-2020 |
| | | | US | 2020179089 A1 | 11-06-2020 |
| | | | US | 2022249214 A1 | 11-08-2022 |
| | | | WO | 2018037318 A1 | 01-03-2018 |
| EP 3858288 | A1 | 04-08-2021 | NONE | | |

EPO FORM P0459

For more details about this annex : see Official Journal of the European Patent Office, No. 12/82